(19) Europäisches Patentamt
European Patent Office
Office européen des brevets

(11) Veröffentlichungsnummer : **0 104 531**
**B1**

(12) # EUROPÄISCHE PATENTSCHRIFT

(45) Veröffentlichungstag der Patentschrift : **21.11.85**

(51) Int. Cl.⁴ : **C 07 C119/045**, C 08 G 18/75

(21) Anmeldenummer : **83108982.6**

(22) Anmeldetag : **12.09.83**

(54) **Gegebenenfalls Isomerengemische darstellende cycloaliphatische Diisocyanate, ein Verfahren zu ihrer Herstellung, sowie ihre Verwendung als Ausgangsmaterialien bei der Herstellung von Polyurethankunststoffen.**

(30) Priorität : **22.09.82 DE 3234996**

(43) Veröffentlichungstag der Anmeldung : **04.04.84 Patentblatt 84/14**

(45) Bekanntmachung des Hinweises auf die Patenterteilung : **21.11.85 Patentblatt 85/47**

(84) Benannte Vertragsstaaten : **BE DE FR GB IT**

(56) Entgegenhaltungen :
**EP-A- 0 024 665**
**EP-A- 0 046 917**
**DE-A- 1 768 832**
**DE-A- 2 361 986**
**Houben-Weyl, Methoden der organischen Chemie, Bd. 111(1950), S. 356-357**
**Bayer-Kunststoffe, 3e Ausgabe (1963) 543**
**Beilsteins Handbuch der organischen Chemie, 4e Aufl, (1930), Bd 13, S. 238, 254**

(73) Patentinhaber : **BAYER AG**
**Konzernverwaltung RP Patentabteilung**
**D-5090 Leverkusen 1 Bayerwerk (DE)**

(72) Erfinder : **Knöfel, Hartmut, Dr.**
**Dülmener Weg 21**
**D-5068 Odenthal-Erberich (DE)**
Erfinder : **Brockelt, Michael**
**Neuenhauser Weg 1**
**D-5060 Bergisch-Gladbach 2 (DE)**
Erfinder : **Penninger, Stefan, Dr.**
**Pommernallee 5**
**D-4047 Dormagen 1 (DE)**
Erfinder : **Stutz, Herbert, Dr.**
**Schulstrasse 81**
**D-4047 Dormagen 5 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 104 531 B1**

Jouve, 18, rue St-Denis, 75001 Paris, France

**Beschreibung**

Die vorliegende Erfindung betrifft neue, nur an einem Cyclohexanring monosubstituierte Methylen-bis-(cyclohexylisocyanate), ein Verfahren zu ihrer Herstellung durch Phosgenierung der ihnen zugrundeliegenden Diamine bzw. Diamingemische und ihre Verwendung als Ausgangsmaterial zur Herstellung von Polyurethankunststoffen nach dem Isocyanat-Polyadditionsverfahren.

Aliphatische oder cycloaliphatische Diisocyanate, wie Hexamethylendiisocyanat, 3,5,5-Trimethyl-1-isocyanato-3-isocyanatomethyl-cyclohexan und 4,4'-Methylen-bis-(cyclohexylisocyanat) sowie deren Stellungs- und/oder Stereoisomerengemische werden in der Polyurethanchemie z. B. zur Herstellung von lichtechten Beschichtungsmaterialien mit guter Witterungsbeständigkeit verwendet. Letztere lassen sich außerdem durch Umsetzung mit Polyolen gut zu Lackbindemitteln, Elastomeren oder Schaumstoffen verarbeiten, falls das Diisocyanat bei Raumtemperatur flüssig und mit den Polyolen verträglich bzw. ausreichend mischbar ist (vgl. z. B. DE-OS 1 768 832). Reines trans, trans-4,4'-Methylen-bis-(cyclohexylisocyanat) ist beispielsweise für diese Zwecke nicht geeignet, da es bei Raumtemperatur fest ist (Schmp.: 83 °C), eine geringe Löslichkeit in Polyolen aufweist und sich deshalb vor Beendigung der Polyadditionsreaktion durch Kristallisation dem Reaktionsgemisch entzieht. Auch das aus trans, trans-, cis, trans- und cis, cis-4,4'-Methylen-bis-(cyclohexylisocyanat) bestehende Stereoisomerengemisch, wie es nach Kernhydrierung von 4,4'-Diaminodiphenylmethan und anschließender Phosgenierung anfällt, ist bei Raumtemperatur fest, da bei der Hydrierung unter normalen Bedingungen das trans, trans-Diamin als Hauptprodukt anfällt, und somit zur Herstellung der obengenannten Polyurethane nur bedingt einsetzbar.

Die Abtrennung von trans, trans-4,4'-Methylen-bis-(cyclohexylisocyanat) aus dem Stereoisomerengemisch ist zwar technisch möglich z. B. durch Fällung der trans, trans-Isomere als Carbamidsäurechlorid und anschließende Filtration (vgl. JA-PS 119 844), jedoch wird durch diesen zusätzlichen Verfahrensschritt die Isocyanatausbeute vermindert, was als großer Nachteil anzusehen ist.

Flüssige cycloaliphatische Diisocyanate können nach dem Stand der Technik durch Phosgenierung von 2,4'-Methylen-bis-(cyclohexylisocyanat) hergestellt werden (vgl. DE-OS 1 768 832). Dieser Offenlegungsschrift zufolge sind Gemische von 2,4'- und 4,4'-Isomeren auch dann flüssig, wenn der 2,4'-Isomerenanteil 30 bis 95 Gew.-% beträgt und das 4,4'-Isomere einen trans, trans-Isomerenanteil von weniger als 50 Gew.-% besitzt. Es erwies sich jedoch als Nachteil, daß das 2,4'-Isomere in reiner Form nur schwer zugänglich ist, da es in drei Stufen durch Kondensation von Anilin mit Formaldehyd, Kernhydrierung und Phosgenierung hergestellt wird und beim Kondensationsschritt 2,4'-Diamino-diphenylmethan selbst unter optimalen Bedingungen nur mit ca. 30 % der Theorie anfällt (vgl. DE-OS 1 937 685), d. h. zur Isolierung des reinen 2,4'-Isomeren ist eine technisch aufwendige Isomerentrennung vonnöten.

Die direkte Herstellung eines 2,4'-isomerenreichen Methylen-bis-(cyclohexylisocyanats) durch Phosgenierung eines entsprechenden Methylen-bis-(cyclohexylamin)-Isomerengemisches ist ebenfalls mit Nachteilen behaftet, da bei der Anilin/Formaldehyd-Kondensation der Gehalt des 2,4'-Diaminodiphenylmethans mit dem des 2,2'-Isomeren korelliert und letzteres bei der Kernhydrierung einer Zersetzungsreaktion unterliegt.

Es war deshalb die Aufgabe der vorliegenden Erfindung neue cycloaliphatische Diisocyanate auf Basis von Methylen-bis-(cyclohexylisocyanat) aufzufinden, die bei Raumtemperatur flüssig sind, in Bezug auf Löslichkeit und Verträglichkeit mit Polyolen den Forderungen der Praxis genügen, und die nicht mit den Nachteilen des Standes der Technik behaftet sind.

Diese Aufgabe konnte überraschenderweise durch die Bereitstellung der nachfolgend näher beschriebenen Diisocyanate bzw. Diisocyanatgemische und des zu ihrer Herstellung geeigneten Verfahrens gelöst werden.

Gegenstand der vorliegenden Erfindung sind, gegebenenfalls Isomerengemische darstellende und gegebenenfalls im Gemisch mit bis zu 40 Gew.-%, bezogen auf Gesamtgemisch, an anderen, gegebenenfalls $C_1$-$C_{12}$-monoalkylsubstituierten Methylen-bis-(cyclohexylisocyanat)-Isomeren vorliegende Diisocyanate der allgemeinen Formel

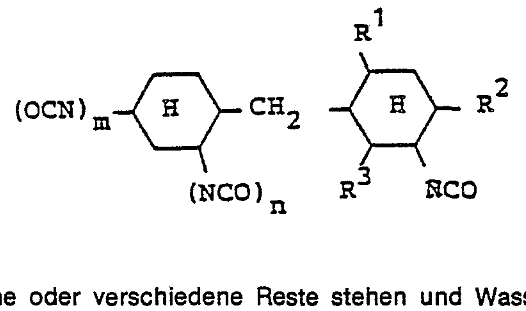

in welcher

$R^1$, $R^2$ und $R^3$ für gleiche oder verschiedene Reste stehen und Wasserstoff oder einen (gegebenenfalls verzweigten) $C_1$-$C_{12}$-Alkylrest bedeuten, mit der Maßgabe, daß zwei der genannten Reste für Wasserstoff und einer der genannten Reste für einen Alkylrest der genannten Art steht, und

n und m jeweils für 0 oder 1 stehen, mit der Maßgabe, daß die Summe m + n den Wert 1 ergibt, wobei

im Falle von m oder n = 0 die verbleibende freie Valenz durch Wasserstoff abgesättigt ist.

Gegenstand der Erfindung ist auch ein Verfahren zur Herstellung dieser Diisocyanate oder Diisocyanatgemische, welches dadurch gekennzeichnet ist, daß man gegebenenfalls als Stellungs- und/oder Stereoisomerengemisch und gegebenenfalls im Gemisch mit bis zu 40 Gew.-%, bezogen auf Gesamtgemisch, an anderen $C_1$-$C_{12}$-monoalkylsubstituierten Methylen-bis-(cyclohexylamin)-Isomeren vorliegende Diamine der Formel

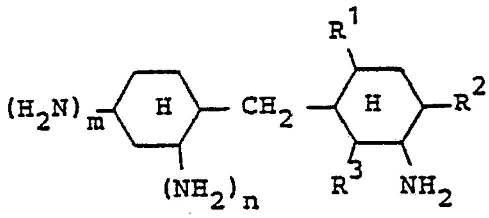

in welcher $R^1$, $R^2$, $R^3$, m und n die obengenannte Bedeutung haben, bei −20 °C bis +250 °C phosgeniert.

Gegenstand der Erfindung ist schließlich auch die Verwendung der neuen Diisocyanate als Aufbaukomponente bei der Herstellung von Polyurethankunststoffen nach dem Isocyanat-Polyadditionsverfahren.

Ausgangsmaterialien für das erfindungsgemäße Verfahren zur Herstellung der neuen Diisocyanate sind, gegebenenfalls als Stellungs- und/oder Stereoisomerengemische und gegebenenfalls im Gemisch mit bis zu 40 Gew.-%, bezogen auf Gesamtgewicht, an anderen $C_1$-$C_{12}$-monoalkylsubstituierten Methylen-bis-(cyclohexylamin)-Isomeren vorliegende Diamine der Formel

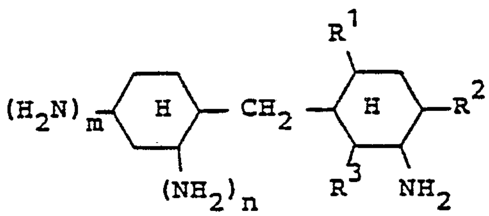

in welcher $R^1$, $R^2$, $R^3$, m und n die bereits obengenannte Bedeutung haben, wobei jedoch einer der Reste $R^1$, $R^2$ oder $R^3$ vorzugsweise für einen $C_1$-$C_4$-Alkylrest, insbesondere einen Methylrest steht.

Dementsprechend handelt es sich bei den erfindungsgemäßen neuen Diisocyanaten vorzugsweise um solche, deren Alkylrest 1 bis 4 Kohlenstoffatome, vorzugsweise 1 Kohlenstoffatom (Methylgruppe) aufweist.

Besonders gut als Ausgangsmaterialien für das erfindungsgemäße Verfahren zur Herstellung von Diisocyanaten bzw. Diisocyanatgemischen einer entsprechenden Isomerenverteilung geeignete Diamine sind, gegebenenfalls als Stellungs- und/oder Stereoisomerengemische, gegebenenfalls mit bis zu 40 Gew.-%, bezogen auf Gesamtgemisch an Diaminen der Formel

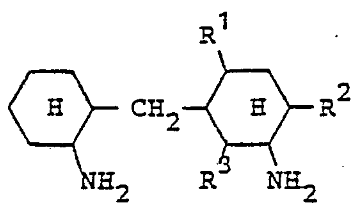

und gegebenenfalls mit bis zu 40, vorzugsweise bis zu 30 Gew.-%, bezogen auf Gesamtgewicht, an anderen $C_1$-$C_{12}$-monoalkylsubstituierten Methylen-bis-(cyclohexylamin)-Isomeren vorliegende Diamine der Formel

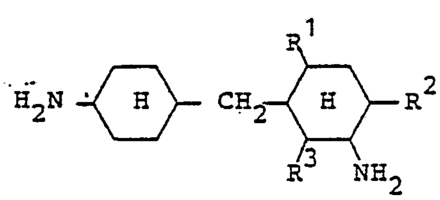

wobei in diesen Formeln die Reste $R^1$, $R^2$ und $R^3$ die obengenannte Bedeutung bzw. bevorzugte Bedeutung haben.

Grundsätzlich kann gesagt werden, daß zu den bevorzugten Ausgangsmaterialien beliebige Diamine der zuletzt genannten beiden allgemeinen Formeln oder beliebige Diamingemische, deren Hauptkomponente bzw. deren Hauptkomponenten diesen Formeln entsprechen, gehören, wobei einer der Reste $R^1$, $R^2$ oder $R^3$ für einen Alkylrest mit 1-4 Kohlenstoffatomen, insbesondere einen Methylrest und die verbleibenden der genannten Reste für Wasserstoff stehen. Zu den bevorzugten bzw. besonders bevorzugten Ausgangsmaterialien gehören daher z. B. 3,4'-Diamino-4-methyldicyclohexylmethan, 3,2'-Diamino-4-methyldicyclohexylmethan, 5,4'-Diamino-2-methyldicyclohexylmethan, 5,2'-Diamino-2-methyldicyclohexylmethan, 3,4'-Diamino-2-methyldicyclohexylmethan, 3,2'-Diamino-2-methyldicyclohexylmethan, sowie diese Diamine als wesentliche Komponente enthaltende Isomerengemische mit anderen Stellungsisomeren. Ebenfalls gut geeignet sind die entsprechenden Ethyl-, Propyl- oder Butylsubstituierten Diaminodicyclohexylmethane bzw. die diese oder ihre Gemische als wesentliche Komponente enthaltenden Isomerengemische mit anderen Stellungsisomeren. Unter « wesentliche Komponente » bzw. « Hauptkomponente » ist im Rahmen der Erfindung ein Anteil der Diamingemische an den besagten Verbindungen von mindestens 60 Gew.-% zu verstehen.

Die Herstellung der beim erfindungsgemäßen Verfahren einzusetzenden Diamine bzw. Diamingemische erfolgt durch katalytische Kernhydrierung der ihnen zugrundeliegenden aromatischen Diamine oder durch zweistufige katalytische Hydrierung der ihnen zugrundeliegenden Dinitroverbindungen, welche die Vorstufe der aromatischen Diamine darstellen. Die Herstellung dieser aromatischen Vorstufen ist beispielsweise in den veröffentlichten europäischen Patentanmeldungen 0024665 bzw. 0046556 beschrieben, bzw. kann in Analogie zu den Verfahrensweisen dieser Veröffentlichungen erfolgen. So erfolgt beispielsweise die Herstellung der oben erwähnten reinen Stellungsisomeren bzw. Diamingemische, die diese Isomeren als Hauptbestandteil enthalten, durch Kondensation von p-Nitrobenzylchlorid mit p- oder o-Nitroalkylbenzolen und anschließende 2-stufige Hydrierung der aromatischen Dinitroverbindungen oder durch Kondensation von o-Nitrobenzoylchlorid mit p- oder o-Nitroalkylbenzolen, Clemmensen-Reduktion und anschließende Kernhydrierung der als Zwischenstufe anfallenden aromatischen Diamine.

Die Kernhydrierung der aromatischen Diamine wird im allgemeinen nach den bekannten Methoden des Standes der Technik (vgl. R. Rylander, Catalytic Hydrogenation in Organic Syntheses, Academic Press New York, San Francisco, London (1979) S. 190). Hierbei werden die aromatischen Diamine bis zur vollständigen Wasserstoffaufnahme katalytisch hydriert. Die Hydrierung erfolgt bei 20 bis 300 °C unter einem Druck von 20 bis 300 bar, vorzugsweise bei einer Temperatur von 100 bis 300 °C, insbesondere von 150 bis 250 °C und bei einem Druck von 70 bis 300 bar, insbesondere 120 bis 250 bar.

Die Hydrierung erfolgt in Gegenwart von 0,1 bis 30 Gew.-%, vorzugsweise von 0,1 bis 10 Gew.-%, bezogen auf katalytisch wirksames Metall einerseits und Diaminoverbindung andererseits, eines Hydrierungskatalysators. Geeignete Katalysatoren sind beispielsweise, gegebenenfalls auf inerten Trägern wie Aktivkohle, Kieselgel und insbesondere Aluminiumoxid vorliegende Elemente der 8. Nebengruppe des Periodensystems der Elemente oder katalytisch wirksame anorganische Verbindungen dieser Elemente. Besonders gut geeignet sind z. B. Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rutheniumverbindungen eingesetzt. Beispiele geeigneter Rutheniumverbindungen sind Rutheniumdioxid, Rutheniumtetroxid, Bariumperruthenit, Natrium-, Kalium-, Silber-, Calcium- oder Magnesiumruthenat, Natriumperruthenat, Rutheniumpentafluorid, Rutheniumtetrafluoridhydrat oder Rutheniumtrichlorid. Falls Trägersubstanzen für die Katalysatoren mitverwendet werden, beträgt der Metallgehalt des Trägerkatalysators im allgemeinen 1 bis 10 Gew.-%, vorzugsweise 1 bis 5 Gew.-%. Im übrigen ist selbstverständlich die Art und Menge des einzusetzenden Katalysators keineswegs wesentlich.

Es ist oft zweckmäßig, die Hydrierungsreaktion in Gegenwart von Ammoniak durchzuführen, da Ammoniak unerwünschte Desaminierungsreaktionen und die Bildung von sekundären Aminen als Nebenprodukte unterdrückt. Falls Ammoniak mitverwendet wird, geschieht dies in Mengen von 0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige aromatische Diamine in Substanz, hochschmelzende Diamine dagegen in gelöster Form hydriert. Als Lösungsmittel eignen sich unter den Reaktionsbedingungen inerte organische Verbindungen mit niedrigem Siedepunkt, vorzugsweise Alkohole wie Methanol, Ethanol, n-Propanol, i-Propanol oder Ether wie z. B. Dioxan, Tetrahydrofuran oder Diethylether oder Kohlenwasserstoffe wie Cyclohexan. Die Durchführung der Hydrierung findet kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls einem Lösungsmittel beschickt, mehrmals mit Inertgas spült und gegebenenfalls Ammoniak zudosiert. Danach drückt man Wasserstoff auf, bringt das Gemisch auf Reaktionstemperatur, hydriert bis Druckkonstanz erreicht ist und rührt während eines weiteren Zeitraums von ca. 0,5 bis 5 Stunden bei gleicher Temperatur. Nach Abkühlung des Reaktionsgemisches und Abtrennung des Katalysators wird das Hydrierungsprodukt im allgemeinen destillativ aufgearbeitet.

Die Hydrierungsprodukte fallen mit hohen Ausbeuten, die im allgemeinen über 90 % der Theorie

liegen, an und können durch Destillation von Nebenprodukten wie z. B. Amino-alkylbenzyl-cyclohexylamin befreit werden. Sie stellen oft Stellungsisomerengemische dar und entsprechen bezüglich der Stellungsisomerie weitgehend den Ausgangsmaterialien. Außerdem liegen die cycloaliphatischen Diamine im allgemeinen als Stereoisomerengemische vor, die, bedingt durch die asymmetrische Substitution des Diamino-dicyclohexylmethan-Grundkörpers an nur einem Cyclohexylring, im allgemeinen bei Raumtemperatur flüssig sind. Eine Auftrennung in einzelne Stellungs- und/oder Stereoisomere ist im allgemeinen bezüglich der technischen Verwendbarkeit der Hydrierungsprodukte als Ausgangsmaterialien für das erfindungsgemäße Verfahren nicht erforderlich, da es hierbei keineswegs auf Isomerenreinheit ankommt und im Gegenteil häufig Isomerengemische erwünscht sind, da dies die Eigenschaften der Diisocyanate verbessert.

Die katalytische Hydrierung der aromatischen Nitroverbindungen wird im allgemeinen zweistufig gemäß US-PS 2 606 925, vorzugsweise in einem niedrigsiedenden Alkohol wie z. B. Methanol, Ethanol oder Isopropanol und in Gegenwart von 0,1 bis 10 Gew.-% eines handelsüblichen Ruthenium-Aluminiumoxid-Trägerkatalysators mit 5-10 Gew.-% Rutheniumgehalt durchgeführt. Im allgemeinen findet die Reduktion der Nitrogruppe zunächst innerhalb des Temperaturbereichs von 20 bis 100 °C unter einem Druck von 1 bis 150 bar statt, während die anschließende Kernhydrierung unter den obengenannten Bedingungen erfolgt.

Bei der Durchführung des erfindungsgemäßen Verfahrens zur Herstellung der neuen Diisocyanate erfolgt die Phosgenierung der beisphelhaft genannten Diamine oder deren Salze nach an sich bekannten Verfahren in Gegenwart eines inerten organischen Lösungsmittels (vgl. Houben-Weyl, Methoden der organischen Chemie, Georg Thieme Verlag Stuttgart (1952), Band 8, 4. Auflage, Seiten 120 ff).

Als zu phosgenierende Salze eignen sich vorzugsweise die Hydrochloride oder Ammoniumcarbamate, die durch Sättigung der Diaminlösungen mit gasförmigen Chlorwasserstoff bzw. Kohlendioxid anfallen. Prinzipiell können auch andere Salze, die beispielsweise durch Neutralisation der Diamine mit Protonen abspaltenden Säuren anfallen, phosgeniert werden.

Die Selektivität der Phosgenierungsreaktion ist weitgehend von der Aminkonzentration und vom Phosgenüberschuß abhängig. Vorzugsweise wird das Phosgen in einem hohen molaren Überschuß und das zu phosgenierende Diamin in stark verdünnter Form eingesetzt. Im allgemeinen beträgt der molare Phosgenüberschuß 100 bis 2 000 %, vorzugsweise 100 bis 1 000 % und die Aminkonzentration in der zur Phosgenierung gelangenden Aminlösung 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60-250 °C, d. h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphthalin und Dichlorethan genannt.

Die Reaktion wird entweder einstufig durch Heißphosgenierung bei Temperaturen von 100 bis 250 °C oder zweistufig durch Kalt/Heißphosgenierung bei Temperaturen von − 20 °C bis 250 °C unter Normaldruck durchgeführt.

Bei Verwendung der freien Amine als Ausgangsverbindung (Basenphosgenierung) wird zuerst bei Temperaturen von − 20 bis + 60 °C Ammoniumcarbamidsäurechlorid hergestellt, das dann bei Temperaturen von 20 bis 250 °C mit Phosgen zum Diisocyanat weiterreagiert.

Eine bevorzugte Ausführungsform des Verfahrens besteht darin, die Amine in einem geeigneten organischen Lösungsmittel zu lösen, diese durch Einleiten von Kohlendioxid als Ammoniumcarbamate zu fällen und die Suspension bei 0-50 °C mit der theoretischen Menge Phosgen zu behandeln. Daraufhin wird die Temperatur unter weiterem Einleiten von Phosgen langsam gesteigert bis bei Temperaturen von 100 bis 180 °C eine klare Lösung entsteht.

Die Reinigung der Verfahrensprodukte erfolgt nach Entphosgenierung durch Abdampfen des Lösungsmittels und anschließende Destillation unter vermindertem Druck.

Die erfindungsgemäßen Verfahrensprodukte, d. h. die neuen erfindungsgemäßen Diisocyanate fallen in hohen Ausbeuten als farblose, niedrigviskose Flüssigkeiten an und stellen wertvolle Aufbaukomponenten bei der Herstellung von Polyurethankunststoffen nach dem Isocyanat-Polyadditionsverfahren dar. Die Stellungs- und/oder Stereoisomerie der neuen Diisocyanate entspricht der Isomerie der bei der Phosgenierung eingesetzten Diamine. Im allgemeinen ist es nicht erforderlich, die beim erfindungsgemäßen Verfahren anfallenden Gemische in einzelne Stellungs- und/oder Stereoisomere aufzutrennen, da die erfindungsgemäßen Verfahrensprodukte ohne derartige Trennungsoperationen direkt der erfindungsgemäßen Verwendung zugeführt werden können. Hierin ist einer der Hauptvorteile der erfindungsgemäßen Diisocyanate bzw. Diisocyanatgemische gegenüber den unsubstituierten Methylenbis-(cyclohexylisocyanaten) des Standes der Technik zu sehen. Die neuen erfindungsgemäßen Diisocyanate können besonders vorteilhaft zur Herstellung von Polyurethanlacken, Polyurethanelastomeren oder Polyurethanschaumstoffen eingesetzt werden, wobei sie bei den an sich bekannten Verfahren zur Herstellung derartiger Kunststoffe anstelle oder zusammen mit den bislang eingesetzten Polyisocyanaten zum Einsatz gelangen.

Die nachfolgenden Beispiele dienen der weiteren Erläuterung der Erfindung. Alle Prozentangaben beziehen sich, soweit nicht anderslautendes vermerkt, auf Gewichtsprozente. Die Analyse der Isomerenverteilung der Zwischen- und Endprodukte erfolgte gaschromatographisch.

Beispiel 1

a) 250 g (1,18 Mol) 5,4'-Diamino-2-methyldiphenylmethan werden zusammen mit 25 g eines handelsüblichen Rutheniumkatalysators mit 5 % Ruthenium und Aluminiumtrioxid als Träger in einen 0,7 l Edelstahlautoklaven vorgelegt. Der freie Raum oberhalb des Autoklaveninhalts wird dreimal jeweils mit Stickstoff und Wasserstoff gespült und der Autoklav mit Wasserstoff unter einem Druck von 200 bar gesetzt. Daraufhin wird unter Rühren auf 180 °C erhitzt und bei 200 bar hydriert, bis die Wasserstoffabsorption beendet ist. Nach weiterem 2-stündigem Rühren unter Reaktionsbedingungen wird das Reaktionsgemisch abgekühlt, der Autoklav entspannt, das Produkt in Methanol aufgenommen und der Katalysator abfiltriert. Man reinigt ihn durch Waschen mit Methanol, vereinigt die organischen Lösungen und zieht das Lösungsmittel ab. Das Rohprodukt wird durch Flashdestillation bei 0,1 mbar von höhersiedenden Produkten befreit und anschließend durch fraktionierte Destillation über eine Vigreux-Kolonne bei 112 bis 114 °C/0,3 mbar gereinigt. Die Ausbeute an 5,4'-Diamino-2-methyldicyclohexylmethan beträgt 254,9 g (96,5 % der Theorie).

b) 56 g (0,25 Mol) 5,4'-Diamino-2-methyldicyclohexylmethan werden in 700 ml trockenem Chlorbenzol bei Raumtemperatur gelöst und unter gutem Rühren ein Strom trockenen Kohlendioxids eingeleitet. Dabei bildet sich ein Niederschlag und die viskose Suspension erwärmt sich auf 40 °C. Man kühlt auf 30 °C und leitet rasch 100 g Phosgen ein, wobei die Temperatur auf 45 °C steigt. Nun erwärmt man das Reaktionsgemisch im Verlauf von 2 Stunden auf 115 °C und leitet in dieser Zeit noch weitere 300 g Phosgen ein. Es findet eine Viskositätsabnahme statt und der Niederschlag löst sich vollständig auf. Man rührt weitere 3 Stunden bei gleicher Temperatur und leitet 150 g/h Phosgen ein. Danach kocht man unter Einleiten von Stickstoff eine Stunde am Rückfluß, destilliert das Lösungsmittel unter vermindertem Druck ab und reinigt das Rohprodukt durch Destillation bei 160-165 °C/0,2-0,4 mbar. Dabei fallen 59 g (82,7 % der Theorie) 5,4'-Diisocyanato-2-methyl-dicyclohexylmethan mit einem NCO-Gehalt von 29,4 %, einem Gehalt an hydrolysierbarem Chlor von 0,06 % und einer Viskosität bei 25 °C von 50 mPa · s an.

Beispiel 2

Das in diesem Beispiel verwendete aromatische Diamingemisch wird in Analogie zu Beispiel 7 der EP-A-0 024 665 durch Nitrierung von 4-Methylbenzylchlorid, Kondensation mit Benzol, Reinigung durch Umkristallisieren, erneute Nitrierung und Reduktion und destillative Aufarbeitung hergestellt. Das wie nachstehend beschrieben weiterverarbeitete aromatische Diamingemisch weist folgende Zusammensetzung auf :

2,4 % 2,2'-Diamino-4-methyl-diphenylmethan
31,3 % 3,2'-Diamino-4-methyl-diphenylmethan
64,5 % 3,4'-Diamino-4-methyl-diphenylmethan
1,8 % undefinierte Diamine.

a) In Analogie zu Beispiel 1a) werden 250 g (1,18 Mol) des genannten aromatischen Diamingemisches und 25 g Ru-Trägerkatalysator (5 % auf $Al_2O_3$) in einem 0,7 l Rührautoklaven vorgelegt und nach dreimaligem Spülen mit Stickstoff und Wasserstoff mit 25 g Ammoniak versetzt. Danach wird unter Rühren bei 180 °C und 200 bar hydriert. Nach Abtrennung des Katalysators und destillativer Isolierung der Reaktionsprodukte bei 120 bis 122 °C/0,1 mbar werden 246 g (93,2 % der Theorie) eines Gemisches verschiedener Diamino-4-methyldicyclohexylmethan-Isomere gewonnen.

b) 112 g (0,5 Mol) des gemäß Beispiel 2a) hergestellten Diamino-4-methyldicyclohexylmethan-Isomerengemischs werden bei 50 °C in 1,4 l getrocknetem Chlorbenzol gelöst und anschließend unter Rühren trockenes Kohlendioxid bis zur Sättigung eingeleitet. Man kühlt die entstandene Suspension auf 10 °C ab und leitet 200 g Phosgen ein. Danach heizt man langsam unter weiterem Einleitern von 300 g Phosgen auf bis der Feststoff bei 120 °C vollständig in Lösung geht. Nun wird unter gleichen Bedingungen weitere 3 Stunden phosgeniert, anschließend eine Stunde am Rückfluß gekocht und das überschüssige Phosgen mit Stickstoff ausgetrieben. Das Rohprodukt wird aufkonzentriert und bei 158-165 °C/0,1 mbar destilliert. Dabei gehen 124 g (89,3 % der Theorie) eines Gemisches verschiedener Diisocyanato-4-methyldicyclohexylmethan-Isomere mit einem NCO-Gehalt von 30,2 %, einem Gehalt an hydrolysierbarem Chlor von 0,06 % und einer Viskosität bei 25 °C von 40 mPa · s über.

Beispiel 3

a) In Analogie zu Beispiel 10 der EP-A-0 024 665 wird durch Dinitrierung eines Kondensats aus 2-Methylbenzylchlorid und Benzol, anschließende Hydrierung der so erhaltenen Dinitroverbindung, ein Diamingemisch folgender Zusammensetzung hergestellt :

1 % 6,2'-Diamino-2-methyldiphenylmethan
14,5 % eines Isomerengemischs aus 3,2'- und 5,2'-Diamino-2-methyldiphenylmethan
0,9 % 6,3'-Diamino-2-methyldiphenylmethan

7,9 % eines Isomerengemischs aus 4,2'- und 6,4'-Diamino-2-methyldiphenylmethan
75 % eines Isomerengemischs, welches zu mehr als 80 % aus 3,4'- und 5,4'-Diamino-2-methyldiphenylmethan und zum Rest aus anderen Diamino-2-methyldiphenylmethanen besteht und
0,7 % undefinierte Polyamine.

250 g (1,18 Mol) dieses Diamingemischs werden innerhalb von 9,5 Stunden bei 180 °C und 200 bar in Gegenwart von 25 g Ru-Katalysator (5 % Ru auf $Al_2O_3$) und 25 g Ammoniak gemäß Beispiel 1a) hydriert. Nach Entspannen des Autoklaven wird das Produkt in Methanol aufgenommen, der Katalysator durch Filtration abgetrennt und das Lösungsmittel abgedampft. Nach fraktionierter Destillation erhält man 221,5 g eines bei 100-108 °C/0,1 mbar siedenden Gemischs verschiedener Diamino-2-methyl-dicyclohexylmethan-Isomere, das in Spuren desaminiertes Produkt und halbhydriertes Diamino-methyldicyclohexylmethan enthält.

b) Man kondensiert bei 0-8 °C 250 g Phosgen in 700 ml Chlorbenzol und läßt bei dieser Temperatur unter Rühren eine Lösung von 112 g (0,5 Mol) des gemäß a) hergestellten Diamins in 700 ml Chlorbenzol zutropfen. Daraufhin heizt man unter Einleiten von 150 g/h Phosgen auf Rückflußtemperatur und rührt unter gleichen Bedingungen weitere 3 Stunden. Nach Entphosgenierung und Abdestillieren des Lösungsmittels wird das Produkt durch Flashdestillation bei 164-168 °C/0,2-0,3 mbar gereinigt. Man erhält 118,6 g (84,2 % der Theorie) von verschiedenen Diisocyanato-2-methyldicyclohexylmethan-Isomeren (NCO-Gehalt : 29,8 %, Gehalt an hydrolysierbarem Chlor : 0,1 %, Viskosität/25 °C : 45 mPa · s).

## Beispiel 4

a) Ein 0,7 l Rührautoklav wird mit 250 g (1,18 Mol) 3,4'-Diamino-4-methyldiphenylmethan und 25 g $Ru/Al_2O_3$-Katalysator (5 % Ru) beschickt. Man spült dreimal mit Stickstoff und Wasserstoff, dosiert 25 g Ammoniak zu, setzt den Autoklaven mit Wasserstoff unter einen Druck von 200 bar und heizt das Gemisch unter Rühren auf 180 °C. Nach vollständiger Wasserstoffabsorption und weiterem zweistündigem Rühren läßt man auf Raumtemperatur abkühlen und entspannt den Autoklaven. Der Katalysator wird durch Filtration abgetrennt, gewaschen und die vereinigten organischen Phasen werden durch Destillation aufgearbeitet. Dabei werden 250 g (94,6 % der Theorie), Kp : 125-126 °C/0,1 mbar) 3,4'-Diamino-4-methyldicyclohexylmethan gewonnen.

$C_{14}H_{28}N_2$ (224,40)

| | | | |
|---|---|---|---|
| ber. : | C 74,9 % | H 12,6 % | N 12,5 % |
| gef. : | C 74,8 % | H 12,8 % | N 12,3 % |

b) Man löst 175 g (0,78 Mol) 3,4'-Diamino-4-methyldicyclohexylmethan in 2 l trockenem Chlorbenzol und leitet bei 40-45 °C trockenes Kohlendioxid bis zur Sättigung ein. Die entstandene Suspension wird auf ca. 20 °C abgekühlt und unter Rühren und Kühlen 200 g Phosgen eingeleitet, so daß die Temperatur konstant bleibt. Unter weiterem Einleiten von 100 g/h Phosgen wird das Gemisch erwärmt bis sich bei ca. 110 °C der gesamte Niederschlag vollständig löst. Man rührt noch weitere 2 Stunden unter konstanten Bedingungen, entphosgeniert, indem man 1 Stunde unter Einleiten von Stickstoff am Rückfluß kocht und destilliert unter vermindertem Druck das Lösungsmittel ab. Das Rohprodukt wird bei 165 °C/0,5 mbar destilliert, wobei 5,5 g Rückstand und 201 g (93,4 % der Theorie) 3,4'-Diisocyanato-4-methyldicyclohexylmethan mit einem NCO-Gehalt von 30,4 % und einem Gehalt an hydrolysierbarem Chlor von 0,01 % anfallen.

## Beispiel 5

a) Ein 1,3 l Rührautoklav wird mit 500 g (2,36 Mol) des in Beispiel 8c) der EP-A-024 665 beschriebenen, bei 150-200 °C/0,133 mbar siedenden, Diamingemisches beschickt, welches folgende Zusammensetzung besitzt :

34,5 % 3,4'-Diamino-4-methyldiphenylmethan
16,9 % 5,4'-Diamino-2-methyldiphenylmethan
7,2 % 3,4'-Diamino-2-methyldiphenylmethan
15,2 % 3,2'-Diamino-4-methyldiphenylmethan
7,5 % 5,2'-Diamino-2-methyldiphenylmethan
3,2 % 3,2'-Diamino-2-methyldiphenylmethan
15,5 % Diamino-methyldiphenylmethane mit unbekannter Stellungsisomerie.

Nach Zugabe von 50 g eines handelsüblichen Ruthenium-Aluminiumoxid-Trägerkatalysators mit 5 % Rutheniumgehalt und dreimaligem Spülen mit Stickstoff und Wasserstoff werden 50 g Ammoniak zudosiert, 200 bar Wasserstoff aufgedrückt und das Reaktionsgemisch unter Rühren auf 180 °C erhitzt. Man hydriert bei 200 bar bis nach 2 Stunden Druckkonstanz erreicht ist, rührt weitere 2 Stunden nach, läßt auf Raumtemperatur abkühlen und entspannt den Autoklaven. Das in Methanol aufgenommene

Rohprodukt wird durch Filtration vom Katalysator abgetrennt und destillativ gereinigt. Als Ausbeute erhält man 497,6 g (94,2 % der Theorie) eines Gemisches verschiedener Diaminomethyldicyclohexylmethan-Isomeren als bei 110-115 °C/0,133 mbar siedende Hauptfraktion, deren Zusammensetzung weitgehend dem oben beschriebenen aromatischen Diamingemisch entspricht.

b) Man löst 224 g des nach Beispiel 5a) hergestellten Diamingemisches in 2 l trockenem Chlorbenzol und leitet unter Rühren bis zur Sättigung Kohlendioxid ein. Die entstandene Suspension wird auf 20-40 °C gekühlt und anschließend unter Einleiten von 100 g/h Phosgen auf 120 °C erhitzt, wobei ab 100 °C eine klare Lösung entsteht. Nach weiterem zweistündigem Rühren unter gleichen Bedingungen wird die Phosgenzufuhr beendet, und das Reaktionsgemisch entphosgeniert, indem man eine Stunde am Rückfluß kocht und gleichzeitig Stickstoff durchbläst. Das Lösungsmittel wird daraufhin unter vermindertem Druck abdestilliert und das Rohisocyanat durch Destillation bei 145-155 °C/0,1 mbar) gereinigt. Die Ausbeute an Diisocyanatgemisch, das einen NCO-Gehalt von 30,4 %, einen Gehalt an hydrolysierbarem Chlor von 0,05 % besitzt und dessen Isomerenzusammensetzung etwa dem Ausgangsprodukt entspricht, beträgt 262 g (94,9 % der Theorie).

## Beispiel 6

a) In einem 0,7 l Rührautoklaven werden 25 g Ruthenium-Aluminiumoxid-Trägerkatalysator (5 % Ru-Gehalt) und 250 g (1,106 Mol) eines gemäß EP-A 46 556 (Beispiel 5) hergestellten Diaminoethyldiphenylmethan-Isomerengemisches vorgelegt, das folgende Zusammensetzung besitzt:

1,9 % Gemisch aus 2,2'-Diamino-4- und 2,2'-Diamino-6-ethyldiphenylmethan
11,1 % 4,2'-Diamino-2-ethyldiphenylmethan
20,1 % Gemisch aus 3,2'-Diamino-2-, 3,2'-Diamino-4- und 5,2'-Diamino-2-ethyldiphenylmethan
66,1 % Gemisch, das zu mindestens 80 % aus 3,4'-Diamino-4- und 5,4'-Diamino-2-ethyldiphenylmethan besteht und bis zu 20 % andere Diaminoethyldiphenylmethane enthält
0,8 % unbekannte Triamine.

Nach mehrmaligem Spülen mit Stickstoff und Wasserstoff und Zudosieren von 25 g flüssigem Ammoniak wird unter Rühren bei 180 °C und 200 bar bis zur Druckkonstanz hydriert.

Man läßt auf Raumtemperatur abkühlen, entspannt den Autoklaven, löst das Rohprodukt in Methanol auf und saugt den Katalysator ab. Das Lösungsmittel wird abgedampft und man gewinnt 253 g eines Diamingemisches, das gemäß gaschromatographischem Befund zu 91 % aus perhydriertem Ausgangsmaterial besteht.

Nach destillativer Aufarbeitung werden 200 g eines Diamingemisches (Kp.: 115-119 °C/0,1 mbar) gewonnen, das zu 99 % aus Diaminoethyldicyclohexylmethan-Isomeren besteht, deren Isomerenzusammensetzung dem Ausgangsprodukt entspricht.

b) Man löst 160 g des Diamingemisches aus Beispiel 6a) in 2 l wasserfreiem Chlorbenzol und leitet unter Rühren trockenes Kohlendioxid ein, was zu einer Feststoffausfällung und einem Temperaturanstieg auf 40 bis 50 °C führt.

Nun wird bei Temperaturen von max. 10 °C 250 g Phosgen eingeleitet und anschließend unter mäßigem Phosgenstrom auf Rückflußtemperatur erwärmt und 5 Stunden nachphosgeniert. Man entphosgeniert und reinigt das Rohisocyanat durch Destillation. Dabei werden 165 g (85,5 % der Theorie) eines bei 138-145 °C/0,1 mbar siedenden Diisocyanato-ethyldicyclohexylmethan-Gemisches gewonnen, das einen NCO-Gehalt von 28,3 % und eine Viskosität von 48 mPa · s/25 °C aufweist und dessen Isomerenzusammensetzung in etwa der des Ausgangsmaterials entspricht.

**Patentansprüche**

1. Gegebenenfalls Isomerengemische darstellende und gegebenenfalls im Gemisch mit bis zu 40 Gew.-%, bezogen auf Gesamtgemisch, an anderen, gegebenenfalls $C_1$-$C_{12}$-monoalkylsubstituierten Methylen-bis-(cyclohexylisocyanat)-Isomeren vorliegende Diisocyanate der allgemeinen Formel

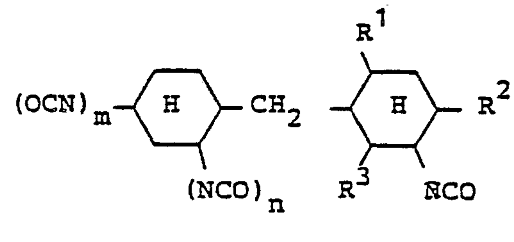

in welcher

$R^1$, $R^2$ und $R^3$ für gleiche oder verschiedene Reste stehen und Wasserstoff oder einen (gegebenenfalls verzweigten) $C_1$-$C_{12}$-Alkylrest bedeuten, mit der Maßgabe, daß zwei der genannten Reste für

Wasserstoff und einer der genannten Reste für einen Alkylrest der genannten Art steht, und

n und m jeweils für 0 oder 1 stehen, mit der Maßgabe, daß die Summe m + n den Wert 1 ergibt, wobei im Falle von m oder n = 0 die verbleibende freie Valenz durch Wasserstoff abgesättigt ist.

2. Diisocyanate gemäß Anspruch 1 der allgemeinen Formel

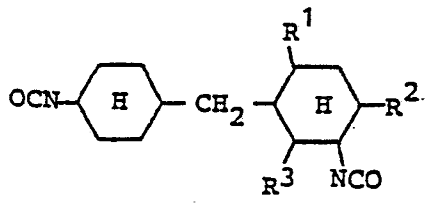

oder Diisocyanatgemische gemäß Anspruch 1, deren Hauptkomponente dieser Formel entspricht, oder deren Hauptkomponenten dieser Formel entsprechen, dadurch gekennzeichnet, daß einer der Reste $R^1$, $R^2$ oder $R^3$ für einen Alkylrest mit 1 bis 4 Kohlenstoffatomen, insbesondere einen Methylrest und die verbleibenden der genannten Reste für Wasserstoff stehen.

3. Diisocyanate gemäß Anspruch 1 der allgemeinen Formel

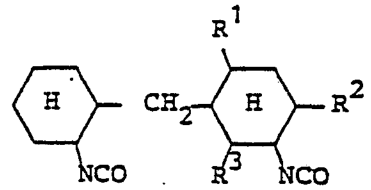

oder Diisocyanatgemische gemäß Anspruch 1, deren Hauptkomponente dieser Formel entspricht, oder deren Hauptkomponenten dieser Formel entsprechen, dadurch gekennzeichnet, daß einer der Reste $R^1$, $R^2$ oder $R^3$ für einen Alkylrest mit 1 bis 4 Kohlenstoffatomen, insbesondere einen Methylrest und die verbleibenden der genannten Reste für Wasserstoff stehen.

4. Verfahren zur Herstellung von Diisocyanaten oder Diisocyanatgemischen gemäß Anspruch 1, dadurch gekennzeichnet, daß man, gegebenenfalls als Stellungs- und/oder Stereoisomerengemisch und gegebenenfalls im Gemisch mit bis zu 40 Gew.-%, bezogen auf Gesamtgemisch, an anderen $C_1$-$C_{12}$-monoalkylsubstituierten Methylen-bis-(cyclohexylamin)-Isomeren vorliegende Diamine der Formel

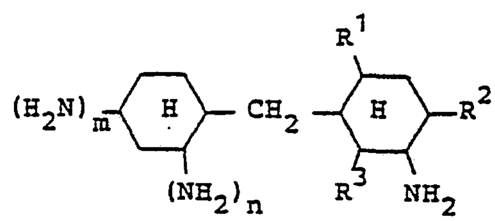

in welcher $R^1$, $R^2$, $R^3$, m und n die in Anspruch 1 genannte Bedeutung haben, bei − 20 °C bis + 250 °C phosgeniert.

5. Verwendung der Diisocyanate bzw. Diisocyanatgemische gemäß Anspruch 1 bis 3 als Aufbaukomponente bei der Herstellung von Polyurethankunststoffen nach dem Isocyanat-Polyadditionsverfahren.

**Claims**

1. Diisocyanates which are optionally in the form of isomer mixtures and are optionally mixed with up to 40 % by weight, based on the total mixture, of other, optionally $C_1$-$C_{12}$-monoalkyl-substituted, methylene-bis-(cyclohexylisocyanate) isomers, and which have the general formula

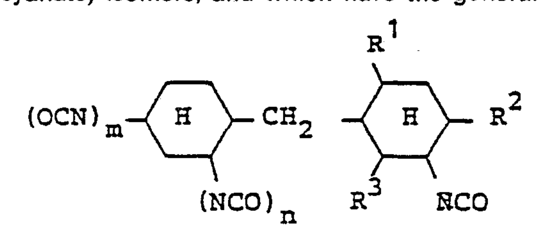

in which

$R^1$, $R^2$ and $R^3$ represent identical or different radicals and denote hydrogen or an (optionally branched) $C_1$-$C_{12}$-alkyl radical, with the proviso that two of the abovementioned radicals represent hydrogen and one of the abovementioned radicals represents an alkyl radical of the abovementioned kind, and

n and m each represent 0 or 1, with the proviso that the sum of m + n equals 1, and in the case where m or n = 0 the remaining free valency is saturated by hydrogen.

2. Diisocyanates according to Claim 1 of the general formula

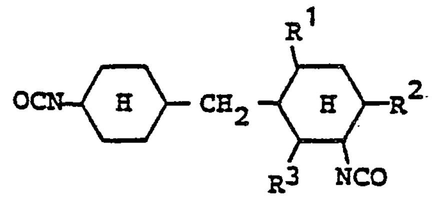

or diisocyanate mixtures according to Claim 1, the main component of which corresponds to this formula, or the main components of which correspond to this formula, characterised in that one of the radicals $R^1$, $R^2$ or $R^3$ represents an alkyl radical with 1 to 4 carbon atoms, in particular a methyl radical and the remaining radicals of those mentioned represent hydrogen.

3. Diisocyanates according to Claim 1 of the general formula

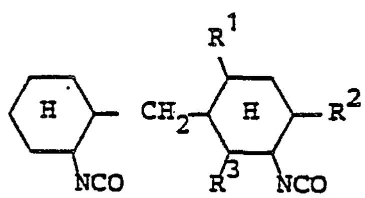

or diisocyanate mixtures according to Claim 1, the main component of which corresponds to this formula, or the main components of which correspond to this formula, characterised in that one of the radicals $R^1$, $R^2$ or $R^3$ represents an alkyl radical with 1 to 4 carbon atoms, in particular a methyl radical and the remaining radicals of those mentioned represent hydrogen.

4. Process for the preparation of diisocyanates or diisocyanate mixtures according to Claim 1, characterised in that diamines which are optionally in the form of a mixture of position and/or stereoisomers and are optionally mixed with up to 40 % by weight, based on the total mixture, of other $C_1$-$C_{12}$-monoalkyl-substituted methylene-bis-(cyclohexylamine) isomers, and which have the formula

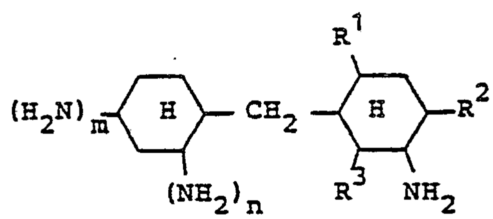

in which $R^1$, $R^2$, $R^3$, m and n have the meaning given in Claim 1, are phosgenated at − 20 °C to + 250 °C.

5. Use of the diisocyanates or diisocyanate mixtures according to Claim 1 to 3 as a starting component in the production of polyurethane plastics by the isocyanate polyaddition process.

**Revendications**

1. Diisocyanates représentant éventuellement des mélanges d'isomères et se présentant éventuellement en mélange avec jusqu'à 40 % en poids (calculé sur le mélange total) d'autres isomères de méthylène-bis-(cyclohexylisocyanate) éventuellement monosubstitués par un groupe alkyle en $C_1$-$C_{12}$, ces diisocyanates répondant à la formule générale :

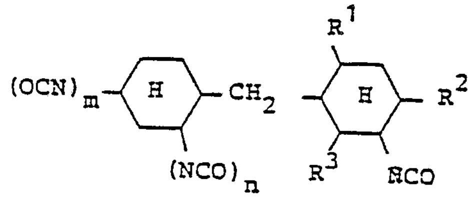

dans laquelle

$R^1$, $R^2$ et $R^3$ sont des radicaux identiques ou différents et représentent chacun un atome d'hydrogène ou un groupe alkyle en $C_1$-$C_{12}$ (éventuellement ramifié), avec cette réserve que deux des radicaux mentionnés représentent chacun un atome d'hydrogène, tandis qu'un de ces radicaux représente un groupe alkyle du type indiqué, et

n et m représentent chacun 0 ou 1, avec cette réserve que la somme m + n a la valeur de 1 et, dans le cas où m ou n = 0, la valence libre restante est saturée par l'hydrogène.

2. Diisocyanates selon la revendication 1, répondant à la formule générale ;

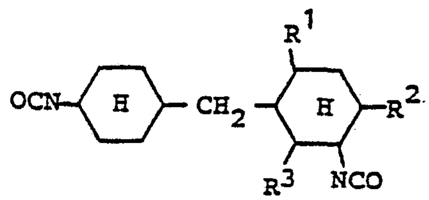

ou mélanges de diisocyanates selon la revendication 1 dont le ou les composants principaux répond ou répondent à cette formule, caractérisés en ce qu'un des radicaux $R^1$, $R^2$ ou $R^3$ représente un groupe alkyle contenant 1 à 4 atomes de carbone, en particulier, un groupe méthyle, tandis que les autres radicaux mentionnés représentent chacun un atome d'hydrogène.

3. Diisocyanates selon la revendication 1, répondant à la formule générale :

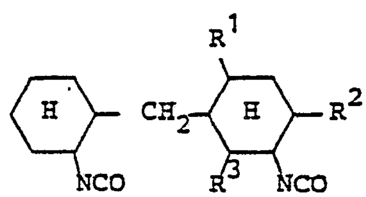

ou mélanges de diisocyanates selon la revendication 1, dont le ou les composants principaux répond ou répondent à cette formule, caractérisés en ce qu'un des radicaux $R^1$, $R^2$ ou $R^3$ représente un groupe alkyle contenant 1 à 4 atomes de carbone, en particulier, un groupe méthyle, tandis que les autres radicaux mentionnés représentent chacun un atome d'hydrogène.

4. Procédé de préparation de diisocyanates ou de mélanges de diisocyanates selon la revendication 1, caractérisé en ce qu'on soumet, à une phosgénation à une température de – 20 °C à + 250 °C, des diamines se présentant éventuellement sous forme d'un mélange d'isomères de position et/ou de stéréoisomères et éventuellement en mélange avec jusqu'à 40 % en poids (calculé sur le mélange total) d'autres isomères de méthylène-bis-(cyclohexylamine) monosubstitués par un groupe alkyle en $C_1$-$C_{12}$, ces diamines répondant à la formule :

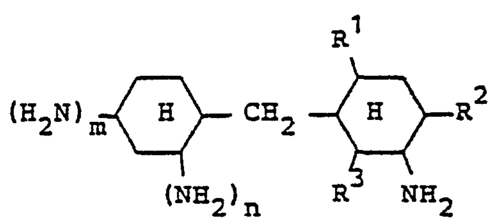

dans laquelle $R^1$, $R^2$, $R^3$, m et n ont les significations indiquées dans la revendication 1.

5. Utilisation des diisocyanates ou des mélanges de diisocyanates selon les revendications 1 à 3 comme composants structuraux lors de la préparation de matières synthétiques de polyuréthanes conformément au procédé de polyaddition d'isocyanates.